# EUROPÄISCHE PATENTSCHRIFT

(11) **EP 0 355 178 B1**
(45) Veröffentlichungstag und Bekanntmachung des Hinweises auf die Patenterteilung: **18.11.1993**
(21) Anmeldenummer: 88113376.3
(22) Anmeldetag: 17.08.1988
(51) Int. Cl.: A61B 17/22

(54) **Einrichtung zum berührungslosen Zertrümmern von Konkrementen im Körper eines Lebewesens**
Apparatus for the contactless desintegration of concrements in a living thing body
Appareil pour la destruction à distance de concrétions dans le corps d'un être vivant

(43) Veröffentlichungstag der Anmeldung: 28.02.1990
(73) Patentinhaber: SIEMENS AKTIENGESELLSCHAFT, 80333 München (DE)
(72) Erfinder: Reichenberger, Helmut, Dipl.-Phys., Dr. rer. nat., D-8501 Eckental (DE); Naser, Georg, Dipl.-Ing. (FH), D-8502 Zirndorf (DE); Schmidt, Erhard, Ing. grad., D-8520 Erlangen (DE)

(56) Entgegenhaltungen:
- EP-A- 0 133 946
- EP-A- 0 148 653
- EP-A- 0 265 742
- EP-A- 0 282 727
- EP-A- 0 332 871
- FR-A- 2 591 467

## Beschreibung

Die Erfindung betrifft eine Einrichtung zum berührungslosen Zertrümmern von Konkrementen im Körper eines Lebewesens mittels fokussierter Schallwellen.

Mittels derartiger Einrichtungen werden in den Körper des zu behandelnden Lebewesens, d.h. eines Patienten, fokussierte Schallwellen eingeleitet, wobei die Einrichtung und der Patient relativ zueinander derart angeordnet sind, daß sich das zu zertrümmernde Konkrement, z.B. ein Nieren- oder Gallenstein, im Fokus der Schallwellen befindet. Unter deren Wirkung zerfällt das Konkrement in Fragmente, die auf natürlichem Wege abgehen oder durch zusätzliche chemotherapeutische Maßnahmen (Litholyse) vollständig oder teilweise aufgelöst werden können.

Aus der US-PS 4 674 505 ist eine Einrichtung der eingangs genannten Art bekannt, die eine Stoßwellenquelle zur Erzeugung von in einer auf der akustischen Achse der Stoßwellenquelle liegenden Fokuszone zusammenlaufenden Stoßwellen und eine Generator- und Steuereinrichtung aufweist, mittels derer die Stoßwellenquelle antreibbar ist. Außerdem sind Mittel zur akustischen Ankopplung der Einrichtung an den Körper des Patienten, mittels derer die Stoßwellen in den Körper des Patienten einleitbar sind, und Mittel zum Verstellen der Einrichtung und des Körpers des Patienten relativ zueinander vorgesehen. Mittels einer derartigen Einrichtung werden bei der Behandlung von Nierensteinleiden gute Erfolge erzielt, ohne daß auf zusätzliche chemotherapeutische Maßnahmen zurückgegriffen werden muß. Eine erhöhte Wirksamkeit der bekannten Einrichtung ist dennoch erwünscht. Dabei soll unter erhöhter Wirksamkeit eine Reduzierung der erforderlichen Anzahl von Stoßwellen bei gleichzeitiger Steigerung des Zerstörungsgrades verstanden werden. Eine erhöhte Wirksamkeit führt also zu einer verkürzten Behandlungsdauer und stellt zugleich sicher, daß die Konkremente in kleinere Fragmente zerfallen, die leichter abgehen können bzw. - falls erforderlich - infolge ihrer vergrößerten Gesamtoberfläche durch chemotherapeutische Maßnahmen rascher aufgelöst werden können. Eine erhöhte Wirksamkeit stellt somit sicher, daß die Belastung des Patienten durch die Behandlung geringer ist, indem einerseits die Dosis akustischer Energie - eine Behandlung mit Stoßwellen ruft Schmerzempfindung, evtl. Hautrötungen und unter Umständen sogar Hämatome hervor - verringert wird und andererseits die medikamentöse Belastung des Patienten durch chemotherapeutische Maßnahmen - diese können über mehrere Monate hinweg erforderlich sein - reduziert oder ausgeschlossen wird.

Es besteht auch die Möglichkeit, bestimmte Gallensteinleiden mit fokussierten Stoßwellen zu therapieren (siehe T. Sauerbruch: "Fragmentation of Gallstones by Extracorporeal Shock Waves", The New England Journal of Medicine, March 27, 1986, Seiten 818 bis 822), jedoch reicht hier infolge der von Nierensteinen abweichenden Beschaffenheit der Gallensteine die Wirksamkeit der bekannten Einrichtung nicht aus, um mit einer dem Patienten zumutbaren Anzahl von Stoßwellen die Steine in einem solchen Grade zu zerstören, daß der Abgang der Fragmente auf natürlichem Wege möglich ist. Es sind daher chemotherapeutische Maßnahmen mit dem Ziele der teilweisen oder vollständigen Auflösung der Fragmente erforderlich. Im Zusammenhang mit der Zertrümmerung von Gallensteinen ist eine verbesserte Wirksamkeit der Einrichtung daher in besonderem Maße wünschenswert, und sei es nur, um chemotherapeutische Maßnahmen im Interesse des Patienten vermeiden oder wenigstens reduzieren zu können.

Der Erfindung liegt die Aufgabe zugrunde, eine Einrichtung der eingangs genannten Art so auszubilden, daß diese eine gegenüber dem Stand der Technik verbesserte Wirksamkeit aufweist, also mit einer reduzierten Anzahl von Stoßwellen einen erhöhten Zerstörungsgrad erreicht, so daß die Belastung des Patienten durch die Behandlung verringert wird und insbesondere chemotherapeutische Maßnahmen überflüssig oder wenigstens nur in reduziertem Umfang erforderlich sind.

Gelöst wird diese Aufgabe nach der Erfindung durch eine Einrichtung zum berührungslosen Zertrümmern von Konkrementen im Körper eines Lebewesens mittels fokussierter Schallwellen, aufweisend eine Stoßwellenquelle zur Erzeugung von in einer auf der akustischen Achse der Stoßwellenquelle liegenden ersten Fokuszone zusammenlaufenden Stoßwellen, eine Ultraschallquelle zur Erzeugung von in einer auf der akustischen Achse der Ultraschallquelle liegenden zweiten Fokuszone zusammenlaufenden Ultraschallwellen, eine Generatoreinrichtung, mittels derer die Stoßwellenquelle und die Ultraschallquelle antreibbar sind, und Mittel zur akustischen Ankopplung der Einrichtung an den Körper des Lebewesens, mittels derer sowohl die Stoßwellen als auch die Ultraschallwellen zur Ausübung von mechanischen Beanspruchungen auf die Konkremente in den Körper des Lebewesens einleitbar sind, wobei die Stoßwellenquelle und die Ultraschallquelle relativ zueinander derart fest angeordnet sind, daß sich ihre akustischen Achsen in einem Schnittpunkt schneiden und die erste und die zweite Fokuszone im wesentlichen zusammenfallen, und wobei mittels der Generatoreinrichtung die Stoßwellenquelle und die Ultraschallquelle sowohl gleichzeitig als auch alternierend zeitlich aufeinanderfolgend antreibbar sind. Die erfindungsgemäße Einrichtung gestattet es also, außer mit fokussierten Stoßwellen auch mit fokussierten Ultraschallwellen auf das Konkrement einzuwirken. Durch die fokussierten Ultraschallwellen wird das Konkrement mechanischen Beanspruchungen unterworfen, die von den durch die Stoßwellen ausgeübten mechanischen Beanspruchungen abweichen. Durch die bei kombinierter Anwendung von Stoßwellen und Ultraschallwellen auftretenden resultierenden mechanischen Beanspruchungen wird eine erhöhte Wirksamkeit der Einrichtung erreicht, d.h. die Anzahl der erforderlichen Stoßwellen wird unter gleichzeitiger Erhöhung des Zerstörungsgrades reduziert. Damit wird die Belastung des Patienten mit akustischer Energie, insbesondere mit Stoßwellen, erheblich reduziert. Infolge des stärkeren Zerstörungsgrades können insbesondere auch Gallenblasensteine so weit zertrümmert werden, daß ein Abgang der Fragmente auf natürlichem Wege möglich ist oder zumindest die erforderlichen chemotherapeutischen Maßnahmen und die damit verbundenen Belastungen für den Patienten erheblich reduziert werden können. Da mittels der erfindungsgemäßen Einrichtung Stoßwellen und Ultraschallwellen sowohl gleichzeitig als auch alternierend zeitlich aufeinanderfolgend erzeugbar sind, ist es möglich, die Art der Behandlung an die Beschaffenheit des jeweils zu zertrümmernden Konkrementes anzupassen. So besteht die Möglichkeit, die Ultraschallquelle derart anzutreiben, daß sie die Ultraschallwellen als Dauerschall abgibt, dem eine Folge von mittels der Stoßwellenquelle erzeugter Stoßwellen überlagert wird. Ein besonders hoher Zerstörungsgrad wird erzielt, wenn die Ultraschallquelle derart angetrieben wird, daß sie die Ultraschallwellen als intermittierenden Dauerschall, also in Form sogenannter Ultraschall-Bursts, abgibt, und die Stoßwellenquelle derart betrieben wird, daß sie während eines Ultraschall-Bursts, insbesondere in der zweiten Hälfte von dessen Zeitdauer, jeweils eine Stoßwelle abgibt oder eine Stoßwelle jeweils unmittelbar an den Ultraschall-Burst anschließend abgibt.

Es wurde zwar bereits versucht, Konkremente, insbesondere Gallensteine, mit Hilfe von Ultraschallwellen zu zertrümmern (E. Craig Coats: "The Application of Ultrasonic Energy to Urinary and Biliary Calculi", The Journal of Urology, Vol. 75, No. 5, May 1956), jedoch wurde keine für die klinische Praxis ausreichende Wirksamkeit erreicht. Neuere Untersuchungen (Francis Frev et al.:"Human Gallstone Dissolution Accelerated by Ultrasound Application", The Journal of the Acoustical Society of America, Supplement 1, Vol. 81, Spring 1987) haben gezeigt, daß Ultraschallwellen allenfalls die Wirksamkeit chemotherapeutischer Maßnahmen unterstützen können. Außerdem sind in den in der EP-A-0 282 727 und der EP-A-0 332 871 nachveröffentlichten prioritätsälteren Patentanmeldungen Geräte zur Behandlung von Steinleiden beschrieben, die sowohl eine Stoßwellenquelle als auch eine Ultraschallquelle enthalten, jedoch ist nicht vorgesehen, daß sich deren akustische Achsen in einem Punkt schneiden und die Fokuszonen der Stoß- und Ultraschallwellen im wesentlichen zusammenfallen.

Gemäß einer bevorzugten Ausführungsform der Erfindung sind die Stoßwellenquelle, die Ultraschallquelle und, falls vorhanden, ein zu einer Ultraschall-Ortungseinrichtung gehöriger Ultraschall-Applikator an einem gemeinsamen Trägerteil angebracht, so daß sich ein kompaktes, leicht zu handhabendes Gerät ergibt, das demzufolge gut am Körper des Patienten applizierbar ist.

Weitere Vorteile und Ausgestaltungen der Erfindung ergeben sich aus der Beschreibung bevorzugter Ausführungsbeispiele anhand der beigefügten Figuren. Es zeigen:
- Fig. 1: den konstruktiven Aufbau einer erfindungsgemäßen Einrichtung in vereinfachter Darstellung in einem Längsschnitt entsprechend der Linie I-I in Fig. 3,
- Fig. 2: in vereinfachter Darstellung einen Längsschnitt entsprechend der Linie II-II in Fig. 3,
- Fig. 3: in vereinfachter Darstellung eine Stirnansicht der Einrichtung,
- Fig. 4: ein Blockschaltbild der zu der Einrichtung nach den Fig. 1 bis 3 gehörigen Generatoreinrichtung,
- Fig. 5: ein Impulsdiagramm der wesentlichen bei der Einrichtung nach den Fig. 1 bis 4 auftretenden Impulse,
- Fig. 6: in vereinfachter Darstellung den konstruktiven Aufbau einer weiteren erfindungsgemäßen Einrichtung in einem Längsschnitt entsprechend der Linie VI-VI in Fig. 7,
- Fig. 7: in vereinfachter Darstellung einen Längsschnitt entsprechend der Linie VII-VII in Fig. 8,
- Fig. 8: eine Stirnansicht der Einrichtung nach Fig. 6 in vereinfachter Darstellung, und
- Fig. 9: ein Blockschaltbild der zu der Einrichtung nach den Fig. 6 und 7 gehörigen Generatoreinrichtung.

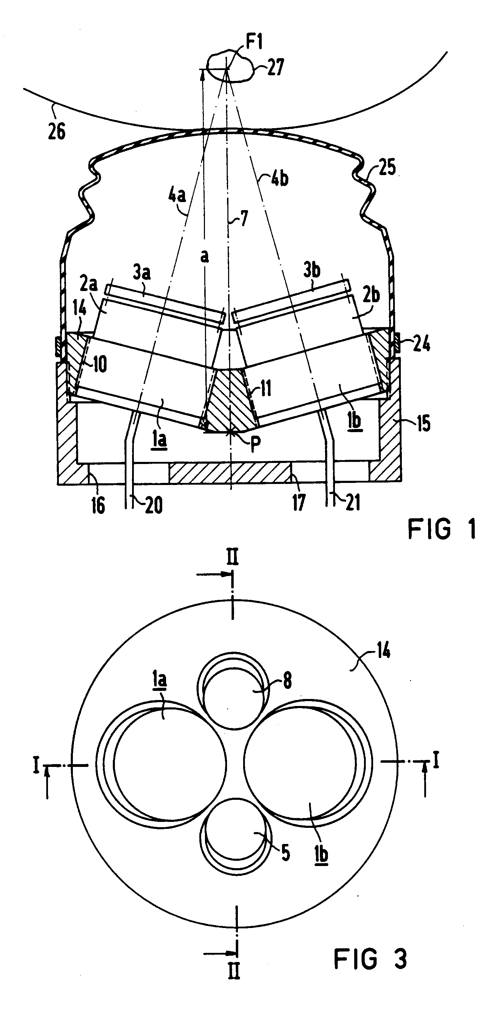

In den Fig. 1 bis 3 ist der konstruktive Aufbau einer erfindungsgemäßen Einrichtung dargestellt, die zwei schematisch dargestellte Stoßwellenquellen 1a, 1b enthält. Bei diesen handelt es sich um Stoßwellenquellen gemäß der US-PS 4 674 505. Die Stoßwellenquellen 1a, 1b weisen also jeweils ein elektro-dynamisches Stoßwellenrohr 2a, 2b mit vorgeschalteter akustischer Sammellinse 3a, 3b auf. Die Stoßwellenquellen 1a, 1b sind somit in der Lage, fokussierte Stoßwellen abzugeben, die jeweils in einer auf der akustischen Achse 4a, 4b der Stoßwellenquellen 1a, 1b liegenden Fokuszone zusammenlaufen. Die Stoßwellenquellen 1a, 1b sind derart gegeneinander geneigt angeordnet, daß sich ihre akustischen Achsen 4a, 4b schneiden und ihre Fokuszonen zusammenfallen. In Fig. 1 ist das Zentrum der Fokuszonen der Stoßwellenquellen 1a, 1b, das dem Schnittpunkt der akustischen Achsen 4a, 4b entspricht, mit F1 bezeichnet.

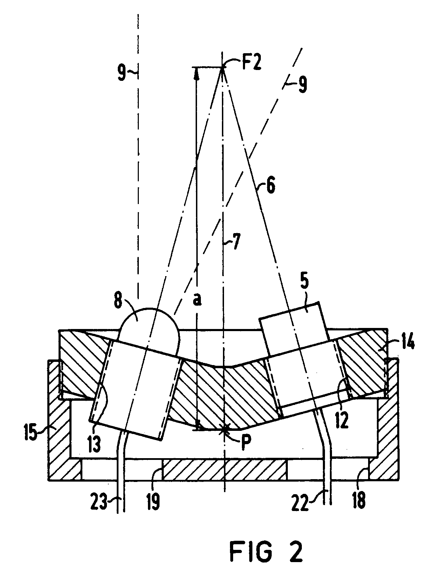

Weiter enthält die erfindungsgemäße Einrichtung eine schematisch dargestellte Ultraschallquelle 5, die in der Lage ist, fokussierte Ultraschallwellen auszusenden, die in einer auf der akustischen Achse 6 der Ultraschallquelle 5 liegenden Fokuszone zusammenlaufen (siehe Fig. 2). Die Ultraschallquelle 5 ist in bezug auf die Stoßwellenquellen 1a, 1b derart angeordnet, daß ihre akustische Achse 6 durch den Schnittpunkt der akustischen Achsen 4a, 4b der Stoßwellenquellen 1a, 1b verläuft und ihre Fokuszone mit den Fokuszonen der Stoßwellenquellen 1a, 1b zusammenfällt. Dies ist in den Fig. 1 und 2 dadurch verdeutlicht, daß der Mittelpunkt F1 der Fokuszonen der Stoßwellenquellen 1a, 1b und der mit F2 bezeichnete Mittelpunkt der Fokuszone der Ultraschallquelle 5 von einem auf der Mittelachse 7 der Einrichtung liegenden Bezugspunkt P jeweils den gleichen Abstand a besitzen.

Außerdem weist die erfindungsgemäße Einrichtung einen ebenfalls schematisch dargestellten zu einer Ultraschall-Ortungseinrichtung gehörigen Ultraschall-Sektor-Applikator 8 auf. Wie aus der Fig. 2 ersichtlich ist, in der der mittels des Ultraschall-Sektor-Applikators 8 abtastbare Sektor 9, der in der Zeichenebene liegt, durch strichlierte Linien angedeutet ist, ist der Ultraschall-Sektor-Applikator 8 derart angeordnet, daß er eine die akustische Achse 6 der Ultraschallquelle 5 enthaltenden Ebene und somit den den Schnittpunkt der akustischen Achsen 4a, 4b und 6 der Stoßwellenquellen 1a, 1b und der Ultraschallquelle 5 umgebenden Bereich abtastet. Hindernisse, z.B. Rippen, für die von der Ultraschallquelle 5 ausgehenden Ultraschallwellen können erkannt werden.

Die Stoßwellenquellen 1a, 1b, die Ultraschallquelle 5 und der Ultraschall-Sektor-Applikator 8 sind in Bohrungen 10, 11, 12, 13 eines gemeinsamen Trägerteiles 14 in der Weise gehaltert, daß die zuvor beschriebene räumliche Zuordnung gewährleistet ist. Dabei liegen sowohl die Stoßwellenquellen 1a und 1b als auch die Ultraschallquelle 5 und der Ultraschall-Sektor-Applikator 8 einander diametral gegenüber. Das Trägerteil 14 ist in einem topfförmigen Gehäuse 15 aufgenommen, dessen Boden mit Durchbrüchen 16, 17, 18, 19 für die zu den Stoßwellenquellen 1a, 1b, der Ultraschallquelle 5 und den Ultraschall-Sektor-Applikator führenden Leitungen 20, 21, 22, 23 versehen ist. Am kreisförmigen Umfang des das Gehäuse 15 geringfügig überragenden Trägerteiles 14 ist mittels eines Spannringes 24 ein flexibler Balg 25 angebracht, mittels dessen die Einrichtung wie in Fig. 1 angedeutet, an den schematisch dargestellten Körper 26 eines Patienten derart anpreßbar ist, daß sich ein zu zertrümmerndes Konkrement 27 im Schnittpunkt der akustischen Achsen 4a, 4b und 6 der Stoßwellenquellen 1a, 1b und der Ultraschallquelle 5 befindet, wobei die entsprechende Position der Einrichtung und des Körpers 26 des Patienten relativ zueinander mittels des Ultraschall-Sektor-Applikators 8 ermittelt wird. Um eine akustische Ankopplung der Einrichtung an den Körper 26 des Patienten zu ermöglichen, also die von den Stoßwellenquellen 1a, 1b ausgehenden Stoßwellen, die von der Ultraschallquelle 5 ausgehenden Ultraschallwellen sowie von dem Ultraschall-Sektor-Applikator 8 ausgehenden Ultraschall-Ortungsimpulse im wesentlichen verlustlos in den Körper 26 des Patienten einleiten zu können, ist der von dem Balg 25 und dem Trägerteil 14 mit den Stoßwellenquellen 1a, 1b, der Ultraschallquelle 5 und dem Ultraschall-Sektor-Applikator 8 begrenzte Raum mit Wasser oder einer anderen Flüssigkeit mit an die akustische Impedanz des Körpers 26 des Patienten angepaßter akustischer Impedanz gefüllt.

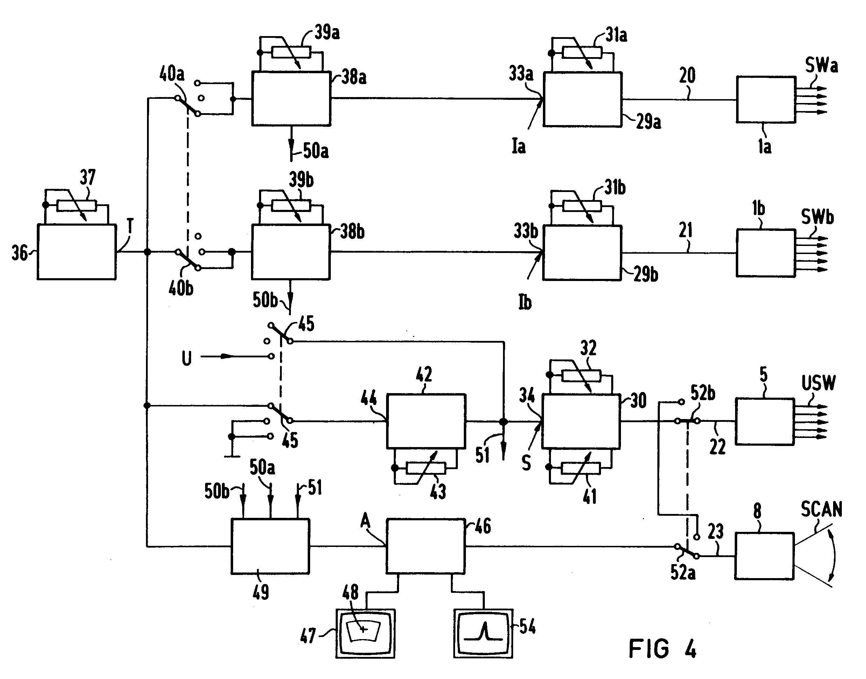

Der Übersichtlichkeit halber wurde übrigens in Fig. 1 auf die Darstellung des Ultraschall-Sektor-Applikators 8, in Fig. 2 auf die Darstellung des Balges 25 mit Spannring 24 und der Stoßwellenquelle 1a und in Fig. 3 auf die Darstellung des Gehäuses 15 und des Balges 25 mit Spannring 24 verzichtet.

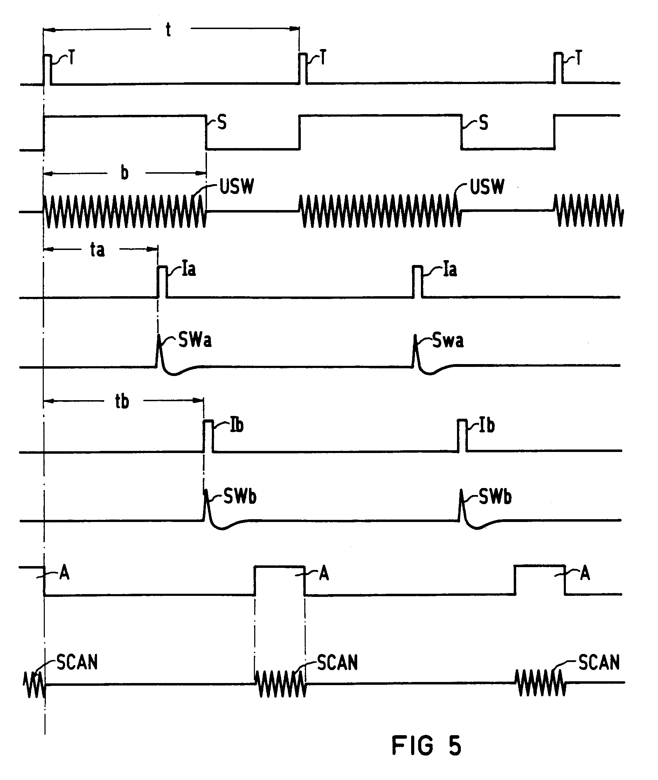

Aus der vorstehenden Beschreibung wird deutlich, daß es mittels der erfindungsgemäßen Einrichtung möglich ist, auf ein zu zertrümmerndes Konkrement 27 sowohl mit von den Stoßwellenquellen 1a, 1b ausgehenden Stoßwellen als auch mit von der Ultraschallquelle 5 ausgehenden Ultraschallwellen einzuwirken und eine Ortung des Konkrementes mittels des Ultraschall-Sektor-Applikators 8 vorzunehmen. Die hierzu erforderliche Generatoreinrichtung 28 ist zusammen mit den jeweils stark schematisch angedeuteten Stoßwellenquellen 1a, 1b, der Ultraschallquelle 5 und dem Ultraschall-Sektor-Applikator 8 in Fig. 4 in Verbindung mit Fig. 5 verdeutlicht. Demnach besitzt die Generatoreinrichtung 28 erste Generatoren 29a, 29b zum Antrieb der Stoßwellenquellen 1a, 1b und einen zweiten Generator 30 zum Antrieb der Ultraschallquelle 5. Der zweite Generator 30 enthält beispielsweise einen Hochfrequenz-Generator, wie er in der US-PS 4 315 514 beschrieben ist. Die ersten Generatoren 29a, 29b enthalten, wie dies in der US-PS 4 674 505 beschrieben ist, im wesentlichen einen Hochspannungskondensator und eine Hochspannungsquelle, mittels derer der Hochspannungsgenerator aufladbar ist. Die Ausgangsleistung der ersten Generatoren 29a, 29b und des zweiten Generators 30 ist zur Beeinflussung der Intensität der erzeugten Stoßwellen SWa, SWb bzw. Ultraschallwellen USW einstellbar. Dies ist durch Einstellwiderstände 31a, 31b und 32 angedeutet. An den zweiten Generator 30 ist außerdem ein Einstellwiederstand 41 angeschlossen, der es gestattet, die Frequenz der von der Ultraschallquelle 5 abgegebenen Ultraschallwellen USW zu verändern. Die ersten Generatoren 29a, 29b besitzen jeweils einen Triggereingang 33a, 33b, über den sie mit einem Triggerimpuls Ia, Ib derart aktivierbar sind, daß sie die Stoßwellenquellen 1a, 1b zur Abgabe einer Stoßwelle SWa, SWb antreiben. Falls die ersten Generatoren 29a, 29b einen Hochspannungskondensator enthalten, kann dies z.B. dadurch erfolgen, daß der aufgeladene Hochspannungskondensator mittels eines geeigneten Hochspannungs-Schaltelementes, das durch den Triggerimpuls Ia, Ib betätigt wird, an die jeweilige Stoßwellenquelle 1a, 1b angeschaltet wird. Der zweite Generator 30 weist einen Steuereingang 34 auf, über den er mittels eines Steuerimpulses S derart aktivierbar ist, daß er für die Impulslänge b des Steuerimpulses S die Ultraschallquelle 5 zur Abgabe von Ultraschallwellen USW antreibt. Dies kann dadurch geschehen, daß der Ausgang des zweiten Generators 30 nur bei Vorliegen eines Steuerimpulses S freigegeben wird.

Die Generatoreinrichtung 28 weist außerdem mit den Generatoren 29a, 29b und 30 zusammenwirkende Steuermittel 35 auf, die dazu dienen, die Triggerimpulse Ia, Ib und den Steuerimpuls S zu erzeugen. Zur Erzeugung der Triggerimpulse Ia, Ib ist ein Impulsgenerator 36 vorhanden, der periodische Impulse T abgibt, deren Periodendauer t einstellbar ist, was durch einen mit dem Impulsgenerator 36 verbundenen Einstellwiderstand 37 angedeutet ist.

Zwischen den Impulsgenerator 36 und die Triggereingänge 33a, 33b der ersten Generatoren 29a, 29b ist jeweils eine Impulsverzögerungsschaltung 38a, 38b geschaltet, die es gestattet, den mittels des Impulsgenerators 36 erzeugten Impuls T um eine Impulsverzögerungszeit ta, tb zu verzögern, bevor dieser als Triggerimpuls Ia, Ib am Triggereingang 33a, 33b der ersten Generatoren 29a, 29b erscheint. Die Impulsverzögerungszeit ta, tb ist einstellbar, was durch mit den Impulsverzögerungsschaltungen 38a, 38b verbundener Einstellwiderstände 39a, 39b schematisch angedeutet ist. Zwischen den Impulsgenerator 36 und den Impulsverzögerungsschaltungen 38a, 38b sind zwei miteinander gekoppelte Umschalter 40a, 40b vorgesehen. Diese gestatten es, in der in Fig. 4 gezeigten Schaltstellung einen Impuls T beiden ersten Generatoren 29a, 29b als Triggerimpuls Ia, Ib zuzuführen. Befinden sich die Umschalter 40a, 40b in ihrer mittleren Schaltstellung, erhält nur der erste Generator 29b einen Triggerimpuls Ib. Nehmen die Umschalter 40a, 40b ihre oberste Schaltposition ein, erhält nur der erste Generator 29a einen Triggerimpuls Ia. Es ist somit möglich, wahlweise einem der ersten Generatoren 29a, 29b oder beiden Triggerimpulse Ia bzw. Ib zuzuführen, wobei die Impulsverzögerungszeiten ta, tb für jeden der ersten Generatoren 29a, 29b individuell einstellbar sind.

Die Steuermittel 35 weisen außerdem einen Steuerimpulsgenerator 42 auf, der den Steuerimpuls S für den Steuereingang 34 des zweiten Generators 30 erzeugt. Dessen Impulslänge 1 ist einstellbar, was durch einen mit dem Steuerimpulsgenerator 42 verbundenen Einstellwiderstand 43 schematisch angedeutet ist. Der Steuerimpulsgenerator 42 besitzt einen Eingang 44, der mit dem Impulsgenerator 36 verbunden ist. Über den Eingang 44 ist der Steuerimpulsgenerator 42 derart aktivierbar, daß er bei Vorliegen eines Impulses T einen Steuerimpuls S abgibt, dessen Impulslänge b von der Stellung des Einstellwiderstandes 43 abhängig ist.

Da somit sowohl die Triggerimpulse Ia, Ib für die ersten Generatoren 29a, 29b als auch der Steuerimpuls S für den zweiten Generator 30 aus dem mittels des Impulsgenerators 36 erzeugten Impuls T abgeleitet sind, liegt eine definierte zeitliche Beziehung zwischen der Abgabe von Stoßwellen SWa mittels der Stoßwellenquelle 1a, der Abgabe von Stoßwellen SWb mittels der Stoßwellenquelle 1b und der Abgabe von Ultraschallwellen USW mittels der Ultraschallquelle 5 vor.

Mittels des zwischen dem Impulsgenerators 36 und dem Steuerimpulsgenerator 42 vorgesehenen doppelpoligen Schalters 45 besteht die Möglichkeit, den Eingang 44 des Steuerimpulsgenerators 42 von dem Impulsgenerator 36 zu trennen und auf Masse zu legen, wenn der Schalter 45 in seine mittlere Schaltposition gebracht wird. Die Abgabe von Ultraschallwellen USW mittels der Ultraschallquelle 5 ist dann unterbunden. Wird der Schalter 45 in seine unterste Position gebracht, liegt der Eingang 44 des Steuerimpulsgenerators 42 weiterhin an Masse, jedoch wird der Steuereingang 34 des zweiten Generators 30 auf ein solches Potential U gelegt, daß der Steuereingang 34 einen Steuerimpuls S "sieht", während der Eingang 44 des Steuerimpulsgenerators 42 weiterhin an Masse liegt. Der zweite Generator 30 treibt somit die Ultraschallquelle 5 zur Erzeugung von Ultraschallwellen USW an, solange sich der Schalter 45 in seiner untersten Schaltposition befindet.

Die Generatoreinrichtung 28 weist weiter eine elektronische Einrichtung 46 zur Erzeugung von Ultraschallbildern auf, die mit dem Ultraschall-Sektor-Applikator 8 in Verbindung steht und an die ein Sichtgerät 47 zur Darstellung des abgetasteten Sektors 9 angeschlossen ist. In das Ultraschallbild des abgetasteten Sektors ist auf dem Sichtgerät 47 ein schematisch angedeutetes Fadenkreuz 48 eingeblendet, das dem Schnittpunkt der akustischen Achsen 4a, 4b und 6 der Stoßwellenquellen 1a, 1b und der Ultraschallquelle 5 entspricht, so daß eine korrekte Positionierung der Einrichtung relativ zu dem Körper des Patienten möglich ist, indem diese in eine solche Position gebracht wird, daß die Abbildung des zu zertrümmernden Konkrementes mit dem Fadenkreuz 48 zusammenfällt. Die elektronische Einrichtung 46 steht mit einer Steuerschaltung 49, die Bestandteil der Steuermittel 35 ist, in Verbindung, die einen die elektronische Einrichtung 46 und den Ultraschall-Sektor-Applikator zur Erzeugung von Ultraschallbildern aktivierenden Aktivierungsimpuls A erzeugt. Die Steuerschaltung 49 steht über eine entsprechende Leitung mit dem Impulsgenerator 36 in Verbindung und erzeugt für jeden von dem Impulsgenerator 36 stammenden Impuls T einen entsprechenden Aktivierungsimpuls A, und zwar zwischen zwei aufeinanderfolgenden Impulsen T jeweils zu einem Zeitpunkt, in dem weder die ersten Generatoren 29a, 29b die Stoßwellenquellen 1a, 1b noch der zweite Generator 30 die Ultraschallquelle 5 antreiben. Der Steuerschaltung 46 ist deshalb über die Leitung 51 der Steuerimpuls S zugeführt. Außerdem steht die Steuerschaltung 46 über Leitungen 50a, 50b mit den Impulsverzögerungsschaltungen 38a, 38b in Verbindung, von denen sie für die Dauer der Impulsverzögerungszeiten ta, tb jeweils ein Signal erhält. Die Steuerschaltung 49 ist derart ausgebildet, daß sie nach Eintreffen eines Impulses T einen Aktivierungsimpuls A dann erzeugt, wenn sie weder über die Leitungen 50a, 50b ein Signal von den Impulsverzögerungsschaltungen 38a, 38b noch über die Leitung 51 einen Steuerimpuls S erhält.

Der Ultraschall-Sektor-Applikator 8 kann mittels eines Umschalters 52a von der elektronischen Einrichtung 46 getrennt werden, indem der Umschalter 52a ausgehend von der in Fig. 4 dargestellten Schaltposition in seine obere Schaltposition gebracht wird. Ein zwischen dem zweiten Generator 30 und der Ultraschallquelle 5 vorgesehener, mit dem Umschalter 52a gekoppelter Umschalter 52b wird gleichzeitig derart betätigt, daß die Ultraschallquelle 5 von dem zweiten Generator 30 getrennt und an die elektronische Einrichtung 46 angeschaltet wird, die dann mit Hilfe der Ultraschallquelle 5 Ultraschall-A-Bilder erzeugt, die auf einem zweiten mit der elektronischen Einrichtung 46 verbundenen Sichtgerät 54 darstellbar sind. Es besteht so die Möglichkeit, neben den mittels des Ultraschall-Sektor-Applikators 8 gewonnenen Informationen zusätzliche Informationen zu erhalten.

In der Fig. 5 wird die Funktionsweise der Einrichtung nach den Fig. 1 bis 4 nochmals deutlicher. Dort sind zunächst die mittels des Impulsgenerators 36 erzeugten Impulse T dargestellt, die eine Periodendauer t aufweisen, die zwischen einer Sekunde und einer Zehntelsekunde einstellbar ist. Aus den Impulsen T werden mittels des Steuerimpulsgenerators 42 Steuerimpulse S gewonnen, deren Impulslänge b zwischen 10 µs und der Impulsdauer t der Impulse T einstellbar ist. Die Steuerimpulse S aktivieren den zweiten Generator 30 derart, daß dieser die Ultraschallquelle 5 zur Abgabe von Ultraschall-Bursts USW antreibt, deren Dauer jeweils der Impulslänge b der Steuerimpulse S entspricht. Die Frequenz der Ultraschall-Bursts ist in nicht dargestellter Weise zwischen 300 kHz und 2 MHz einstellbar. Die Leistungsdichte der Ultraschall-Bursts ist zwischen 1 bis 10 Watt/cm² einstellbar.

Aus den mittels des Impulsgenerators 36 gelieferten Impulsen T werden mittels der Impulsverzögerungsschaltungen 38a, 38b die Triggerimpulse Ia, Ib für die ersten Generatoren 29a, 29b abgeleitet. Diese sind gegenüber dem Impuls T um die Impulsverzögerungszeit ta, tb verzögert, wobei Impulsverzögerungszeiten ta, tb zwischen "O" und der Periodendauer t der Impulse T einstellbar sind. Mittels der Triggerimpulse Ia, Ib werden die ersten Generatoren 29a, 29b derart aktiviert, daß sie die Stoßwellenquellen 1a, 1b bei Vorliegen eines Triggerimpulses Ia, Ib jeweils zur Erzeugung einer Stoßwelle SWa, SWb antreiben. Der Stoßwellen-Spitzendruck ist zwischen 20 und 150 Mpa einstellbar. Im Falle der Fig. 5 erfolgt die Abgabe von Stoßwellen SWa mittels der Stoßwellenquelle 1a jeweils während der zweiten Hälfte der Zeitdauer eines Ultraschall-Bursts USW, während mittels der Stoßwellenquelle 1b eine Stoßwelle SWb jeweils unmittelbar am Ende eines Ultraschall-Bursts USW erzeugt wird. Eine derartige Betriebsweise der erfindungsgemäßen Einrichtung gewährleistet eine besonders hohe Wirksamkeit.

Die mittels der Steuerschaltung 49 aus den Triggerimpulsen Ia, Ib und den Steuerimpulsen S gewonnenen Aktivierungsimpulse A dienen dazu, die mit dem Ultraschall-Sektor-Applikator 8 verbundene elektronische Einrichtung 46 zur Erzeugung von Ultraschallbildern zu aktivieren, worauf der Ultraschall-Sektor-Applikator Ortungsimpulse aussendet und empfängt. Dies ist in den Fig. 4 und 5 durch die Angabe SCAN angedeutet. Wie bereits erwähnt wurde und außerdem aus der Fig. 5 ersichtlich ist, erfolgt die Anfertigung eines Ultraschallbildes jeweils dann, wenn weder die Stoßwellenquellen 1a, 1b noch die Ultraschallquelle 5 aktiv sind, so daß störende Beeinträchtigungen der erzeugten Ultraschallbilder durch die Abgabe von Stoßwellen oder Ultraschallwellen ausgeschlossen sind.

Aus der Beschreibung der Fig. 4 wird deutlich, daß auch von den in Fig. 5 dargestellten Verhältnissen abweichende Betriebsweisen der erfindungsgemäßen Einrichtung möglich sind. So können z.B. die Impulsverzögerungszeiten ta und tb so gewählt werden, daß die Abgabe von Stoßwellen SWa und SWb gleichzeitig erfolgt. Weiter kann die Abgabe von Stoßwellen SWa und SWb zu beliebigen Zeitpunkten innerhalb einer Periode t der Impulse T erfolgen, indem die Impulsverzögerungszeiten ta bzw. tb entsprechend gewählt werden. Es besteht auch die Möglichkeit, nur Stoßwellen SWa oder SWb mittels der Stoßwellenquelle 1a oder der Stoßwellenquelle 1b zu erzeugen, indem die Umschalter 40a, 40b entsprechend betätigt werden. Weiter kann eine Behandlung auch ausschließlich mit Stoßwellen SWa, SWb erfolgen, indem der Umschalter 45 entsprechend betätigt wird. Ebenfalls durch entsprechendes Betätigen des Umschalters 45 ist es möglich, Ultraschallwellen USW in Form von Dauerschall zu erzeugen, wobei sich die Dauerschallabgabe über die gesamte Behandlungsdauer oder einzelne Behandlungsabschnitte erstreckt und dem Dauerschall eine periodische Folge von Stoßwellen SWa, SWb überlagert ist. Es wird somit deutlich, daß die Art der Behandlung an die Beschaffenheit des jeweils zu zertrümmernden Konkrementes optimal anpaßbar ist. Auch die Betriebsweise der Ortungseinrichtung kann den jeweiligen Bedürfnissen angepaßt werden, da die Möglichkeit besteht, die Ultraschallquelle 5 anstelle des Ultraschall-Sektor-Applikators oder abwechselnd mit diesem für Ortungszwecke zu betreiben.

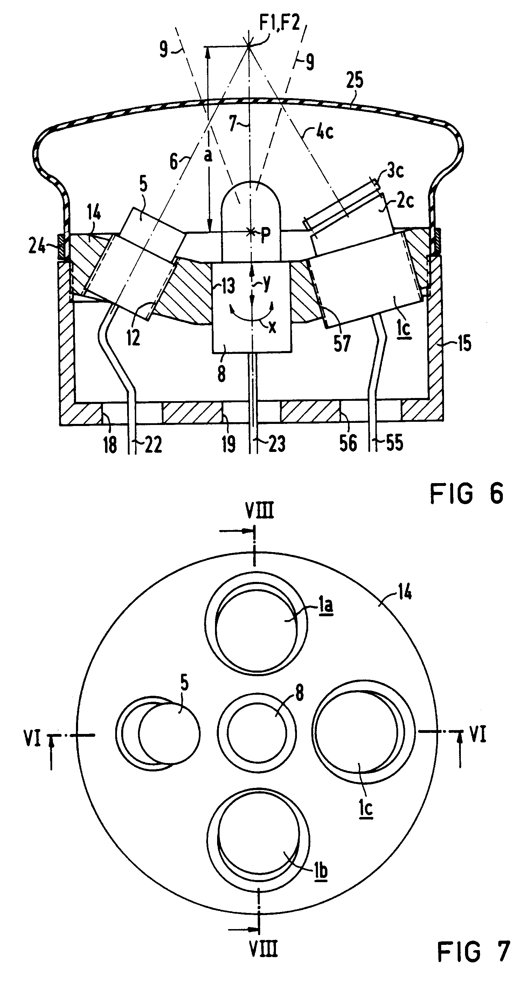

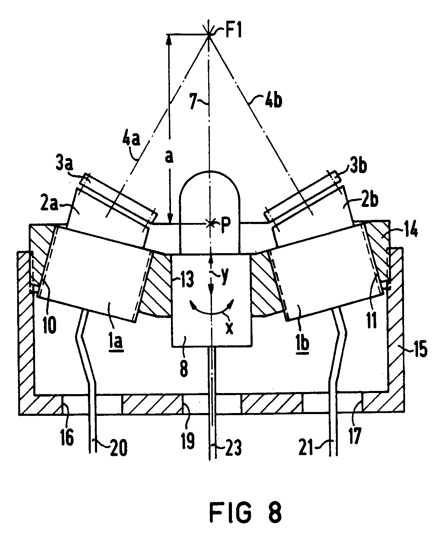

In den Fig. 6 bis 9 ist ein weiteres Ausführungsbeispiel der erfindungsgemäßen Einrichtung dargestellt, das mit dem zuvor beschriebenen weitgehend übereinstimmt, weshalb gleiche Teile gleiche Bezugsziffern tragen. Im Gegensatz zu der zuvor beschriebenen Einrichtung weist die Einrichtung nach den Fig. 6 bis 9 zusätzlich zu den Stoßwellenquellen 1a und 1b eine dritte Stoßwellenquelle 1c auf, die in der Lage ist, fokussierte Stoßwellen SWc abzugeben, die in einer auf der akustischen Achse 4c der Stoßwellenquelle 1c liegenden Fokuszone zusammenlaufen. Zu der Stoßwellenquelle 1c führt eine Leitung 55 durch einen zusätzlichen Durchbruch 56 im Boden des Gehäuses 15. Außerdem ist wieder eine Ultraschallquelle 5 vorhanden, die in der Lage ist, fokussierte Ultraschallwellen USW auszusenden, die in einer auf der akustischen Achse 6 der Ultraschallquelle 5 liegenden Fokuszone zusammenlaufen. Die Stoßwellenquellen 1a, 1b, 1c und die Ultraschallquelle 5 sind wieder in Bohrungen eines gemeinsamen Trägerteiles 14 aufgenommen, und zwar derart, daß die Stoßwellenquellen 1a und 1b einander diametral gegenüberliegend angeordnet sind, während die Ultraschallquelle 5 der Stoßwellenquelle 1c diametral gegenüberliegt. Das Trägerteil 14 weist eine zusätzliche Bohrung 57 zur Aufnahme der Stoßwellenquelle 1c auf. Die akustischen Achsen 4a, 4b, 4c der Stoßwellenquellen 1a, 1b, 1c sind derart gegeneinander geneigt, daß sie sich in einem auf der Mittelachse 7 der Einrichtung liegenden Schnittpunkt schneiden, der den Mittelpunkten der Fokuszonen der Stoßwellenquellen 1a, 1b, 1c entspricht und in den Fig. 6 und 7 mit F1 bezeichnet ist. Die Ultraschallquelle 5 ist derart angeordnet, daß ihre akustische Achse 6 durch den Schnittpunkt der akustischen Achse 4a, 4b, 4c der Stoßwellenquellen 1a, 1b, 1c verläuft und der Mittelpunkt F2 ihrer Fokuszone mit dem Mittelpunkt der Fokuszonen der Stoßwellenquellen 1a, 1b, 1c zusammenfällt. Dies ist in den Fig. 6 und 7 dadurch verdeutlicht, daß die Mittelpunkte F1 und F2 der Fokuszonen der Stoßwellenquellen 1a, 1b, 1c und der Ultraschallquelle 5 von einem auf der Mittelachse 7 der Einrichtung liegenden Bezugspunkt P jeweils den gleichen Abstand a besitzen.

Außerdem ist wieder ein zu einer Ultraschall-Ortungseinrichtung gehöriger Ultraschall-Sektor-Applikator 8 vorgesehen, der dieses Mal in einer zentralen Bohrung des Trägerteiles 14 aufgenommen ist, so daß der mittels des Ultraschall-Sektor-Applikators 8 abtastbare Sektor 9 die Mittelachse 7 der Einrichtung enthält. Der Ultraschall-Sektor-Applikator 8 ist in dem Trägerteil 14 verdrehbar aufgenommen, was durch den Doppelpfeil X angedeutet ist. Es ist so möglich, den Ultraschall-Sektor-Applikator 8 in eine Position zu bringen, in der der Sektor 9, so wie dies in Fig. 6 dargestellt ist, die akustischen Achsen 4c und 6 der Stoßwellenquelle 1c und der Ultraschallquelle 5 enthält. Er kann aber auch in eine Positon gebracht werden, in der der abtastbare Sektor 9 die akustischen Achsen 4a und 4b der Stoßwellenquellen 1a und 1b enthält. Es können somit Hindernisse sowohl für die von der Ultraschallquelle 5 ausgehenden Ultraschallwellen USW und die von der Stoßwellenquelle 1c ausgehenden Stoßwellen SWc als auch für die von den Stoßwellenquellen 1a und 1b ausgehenden Stoßwellen SWa und SWb erkannt werden. Außerdem besteht die Möglichkeit, den Ultraschall-Sektor-Applikator 8 in Richtung der Mittelachse 7 der Einrichtung zu verschieben, wie dies durch den Doppelpfeil Y angedeutet ist.

Am Umfang des in dem Gehäuse 15 aufgenommenen Trägerteiles 14 ist wieder mittels eines Spannringes 24 ein fexibler Balg 25 angebracht, mittels dessen die Einrichtung an den nicht dargestellten Körper eines Patienten anpreßbar ist. Um eine akustische Ankopplung der Einrichtung an den Körper des Patienten zu ermöglich, ist der von dem Balg 25 und dem Trägerteil 14 mit den Stoßwellenquellen 1a, 1b, 1c, der Ultraschallquelle 5 und dem Ultraschall-Sektor-Applikator 8 begrenzte Raum mit Wasser oder dergleichen gefüllt.

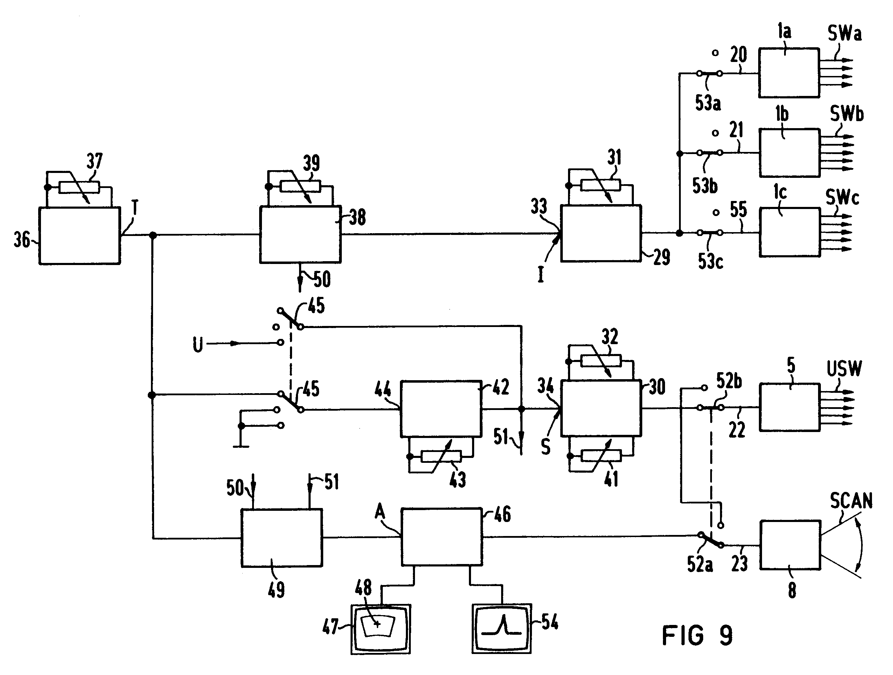

Die zu der Einrichtung nach den Fig. 6 bis 8 gehörige elektrische bzw. elektronische Einrichtung ist zusammen mit den jeweils schematisch angedeuteten Stoßwellen 1a, 1b, 1c, der Ultraschallquelle 5 und dem Ultraschall-Sektor-Applikator 8 in Fig. 9 verdeutlicht. Demnach weist die Generatoreinrichtung 28 im Gegensatz zu dem zuvor beschriebenen Ausführungsbeispiel nur einen einzigen ersten Generator 29 auf, der zum Antrieb der Stoßwellenquellen 1a, 1b, 1c dient. Diese können mit Hilfe von Schaltern 53a, 53b, 53c, wahlweise einzeln oder gemeinsam an den ersten Generator 30 angeschaltet werden. Sind mehrere Stoßwellenquellen 1a, 1b, 1c an den ersten Generator 29 angeschaltet, geben diese somit gleichzeitig Stoßwellen SWa, SWb, SWc ab. Die Ausgangsleistung des ersten Generators 29 ist mittels eines Einstellwiderstandes 31 einstellbar. Der erste Generator 29 weist einen Triggereingang 33 auf, über den er mittels eines Triggerimpulses I derart aktivierbar ist, daß er die angeschalteten Stoßwellenquellen 1a, 1b, 1c zur Abgabe von Stoßwellen SWa, SWb, SWc antreibt.

Die zu der Generatoreinrichtung 28 gehörigen Steuermittel 35 unterscheiden sich von dem zuvor beschriebenen Ausführungsbeispiel insoweit, als nur eine einzige Impulsverzögerungsschaltung 38 vorgesehen ist, deren Impulsverzögerungszeit mittels eines Einstellwiderstandes 39 einstellbar ist. Demgemäß führt neben Leitung 51, über die der Steuerschaltung 49 der Steuerimpuls S zugeführt wird, nur eine einzige Leitung 50 für den Triggerimpuls I zu der Steuerschatlung 49. Bezüglich der weiteren Merkmale wird auf das zuvor beschriebene Ausführungsbeispiel verwiesen.

## Patentansprüche

1. Einrichtung zum Zertrümmern von Konkrementen (27) im Körper (26) eines Lebewesens mittels fokussierter Schallwellen (SWa, SWb, SWc, USW), aufweisend eine Stoßwellenquelle (1a, 1b, 1c) zur Erzeugung von in einer auf der akustischen Achse (4a, 4b, 4c) der Stoßwellenquelle (1a, 1b, 1c) liegenden ersten Fokuszone (F1) zusammenlaufenden Stoßwellen (SWa, SWb, SWc), eine Ultraschallquelle zur Erzeugung von in einer auf der akustischen Achse (6) der Ultraschallquelle (5) liegenden zweiten Fokuszone (F2) zusammenlaufenden Ultraschallwellen (USW), eine Generatoreinrichtung (28), mittels derer die Stoßwellenquelle (1a, 1b, 1c) und die Ultraschallquelle (5) antreibbar sind, und Mittel (25) zur akustischen Ankopplung der Einrichtung an den Körper (26) des Lebewesens, mittels derer sowohl die Stoßwellen (SWa, SWb, SWc) als auch die Ultraschallwellen (USW) zur Ausübung von mechanischen Beanspruchungen auf die Konkremente in den Körper (26) des Lebewesens einleitbar sind, wobei die Stoßwellenquelle (1a, 1b 1c) und die Ultraschallquelle (5) relativ zueinander derart fest angeordnet sind, daß sich ihre akustischen Achsen (4a, 4b, 4c, 6) in einem Schnittpunkt schneiden und die erste und die zweite Fokuszone (F1 bzw. F2) im wesentlichen zusammenfallen, und wobei mittels der Generatoreinrichtung (28) die Stoßwellenquelle (1a, 1b, 1c) und die Ultraschallquelle (5) sowohl gleichzeitig als auch alternierend zeitlich aufeinanderfolgend antreibbar sind.

2. Einrichtung nach Anspruch 1, **dadurch gekennzeichnet,** daß eine Ultraschall-Ortungseinrichtung (5, 8, 46, 47, 52a, 52b, 54) zur Ortung zu zertrümmernder Konkremente (27) vorgesehen ist, die einen akustisch mit dem Körper (26) des Lebewesens koppelbaren Ultraschall-Applikator (5, 8) und mit diesem verbindbare Mittel (46, 47, 54) zur Erzeugung von Ultraschallbildern aufweist, wobei Ultraschallbilder wenigstens des den Schnittpunkt der akustischen Achsen (1a, 1b, 1c und 6) der Stoßwellenquelle (1a, 1b, 1c) und der Ultraschallquelle (5) umgehenden Bereiches erzeubar sind.

3. Einrichtung nach Anspruch 2, **dadurch gekennzeichnet,** daß als Ultraschall-Applikator (8) ein Ultraschall-Sektor-Applikator vorgesehen ist, welcher derart angeordnet ist, daß eine die akustische Achse (4a, 4b, 4c) der Stoßwellenquelle (1a, 1b, 1c) und/oder die akustische Achse (6) der Ultraschallquelle (5) enthaltende Ebene abtastbar ist.

4. Einrichtung nach Anspruch 2 oder 3, **dadurch gekennzeichnet,** daß als Ultraschall-Applikator die Ultraschallquelle (5) vorgesehen ist, die wahlweise an die Mittel (46, 54) zur Erzeugung von Ultraschallbildern oder die Generatoreinrichtung (28) anschaltbar ist.

5. Einrichtung nach einem der Ansprüche 1 bis 4, **dadurch gekennzeichnet,** daß die Stoßwellenquelle (1a, 1b, 1c), die Ultraschallquelle (5) und gegebenenfalls der Ultraschall-Applikator (8) an einem gemeinsamen Trägerteil (14) angebracht sind.

6. Einrichtung nach einem der Ansprüche 1 bis 5, **dadurch gekennzeichnet,** daß die Generatoreinrichtung (28) einen ersten Generator (29, 29a, 29b), mittels dessen die Stoßwellenquelle (1a, 1b, 1c) antreibbar ist, einen zweiten Generator (30), mittels dessen die Ultraschallquelle (5) antreibbar ist, und mit den Generatoren (29, 29a, 29b, 30) zusammenwirkende Steuermittel (35) aufweist, wobei der erste Generator (29, 29a, 29b) einen Triggereingang (33, 33a, 33b) aufweist, über den er mittels eines Triggerimpulses (I, Ia, Ib) derart aktivierbar ist, daß er die Stoßwellenquelle (1a, 1b, 1c) bei Vorliegen eines Triggerimpulses (I, Ia, Ib) zur Abgabe einer Stoßwelle (SWa, SWb, SWc) antreibt, wobei der zweite Generator (30) einen Steuereingang (34) aufweist, über den er mittels eines Steuerimpulses (S) derart aktivierbar ist, daß er für die Impulslänge (b) des Steuerimpulses (S) die Ultraschallquelle (5) zur Abgabe von Ultraschallwellen (USW) antreibt, und wobei die Steuermittel (35) Mittel (36, 39, 39a, 39b) zur Erzeugung des Triggerimpulses (I, Ia, Ib) und Mittel (42, 45, U) zur Erzeugung des Steuerimpulses (S) aufweisen und die Mittel (36, 39, 39a, 39b) zur Erzeugung des Triggerimpulses (I, Ia, Ib) einen Impulsgenerator (36) aufweisen, der periodische Impulse (T) abgibt, deren Periodendauer (t) einstellbar ist.

7. Einrichtung nach Anspruch 6, **dadurch gekennzeichnet,** daß die Mittel (42, 45, U) zur Erzeugung des Steuerimpulses (S) gemäß einer Ausführungsform der Erfindung einen Steuerimpulsgenerator (42) aufweisen, der Steuerimpulse (S) abgibt, deren Impulslänge (b) einstellbar ist, und der einen Eingang (44) aufweist, über den er mittels eines Impulses (T) derart aktivierbar ist, daß er bei Vorliegen eines Impulses (T) einen Steuerimpuls (S) abgibt, und daß die Steuermittel (35) zur Erzeugung des Impulses (T) für den Eingang (44) des Steuerimpulsgenerators (42) einen Impulsgenerator (36) aufweisen, der periodische Impulse (T) erzeugt, deren Periodendauer (t) einstellbar ist.

8. Einrichtung nach Anspruch 7, **dadurch gekennzeichnet,** daß die Mittel (36, 39, 39a, 39b) zur Erzeugung des Triggerimpulses (I, Ia, Ib) und die Mittel (42, 45, U) zur Erzeugung des Steuerimpulses (S) einen gemeinsamen Impulsgenerator (36) aufweisen.

9. Einrichtung nach einem der Ansprüche 6 bis 8, **dadurch gekennzeichnet,** daß die Mittel (36, 39, 39a, 39b) zur Erzeugung des Triggerimpulses (I, Ia, Ib) eine zwischen den Impulsgenerator (36) und den Triggereingang (33, 33a, 33b) des ersten Generators (29, 29a, 29b) geschaltete Impulsverzögerungsschaltung (39, 39a, 39b) zur Verzögerung der von dem Impulsgenerator (36) gelieferten Impulse (T) aufweisen, deren Impulsverzögerungszeit (ta, tb) einstellbar ist.

10. Einrichtung nach einem der Ansprüche 2 bis 9, **dadurch gekennzeichnet,** daß die Steuermittel (35) eine der Ultraschall-Ortungseinrichtung (5, 8, 46, 47, 52a, 52b, 54) zugeordnete Steuerschaltung (49) aufweisen und die Ultraschall-Ortungseinrichtung (5, 8, 46, 47) mittels eines Aktivierungsimpulses (A) zur Erzeugung von Ultraschallbildern aktivierbar ist, wobei die Steuerschaltung (49) den Aktivierungsimpuls (A) erzeugt und derart ausgebildet ist, daß sie einen Aktivierungsimpuls (A) nur dann erzeugt, wenn weder der erste Generator (29, 29a, 29b) die Stoßwellenquelle (1a, 1b, 1c) noch der zweite Generator (30) die Ultraschallquelle (5) antreibt.

## Claims

1. A device for shattering concretions (27) in the body (26) of a living organism by means of focused sound waves (SWa,SWb,SWc,USW) having a shock wave source (1a,1b,1c) for generating shock waves (SWa, SWb, SWc) which converge in a first focus zone (F1) lying on the acoustic axis (4a,4b,4c) of the shock wave source (1a,1b,1c), an ultra sound source for generating ultra sound waves (USW) which converge in a second focus zone (F2) lying on the acoustic axis (6) of the ultra sound source (5), a generator device (28) by means of which the shock wave source (1a,1b,1c) and the ultrasound source (5) are able to be driven and means (25) for acoustic coupling of the device to the body (26) of the living organism, by means of which the shock waves (SWa,SWb,SWc) as well as the ultra sound waves (USW) for applying mechanical stress to the concretions are able to be introduced into the body (26) of the living organism, wherein the shock wave source (1a,1b,1c) and the ultra sound source (5) are arranged closely relative to each other in that their acoustic axes (4a,4b,4c,6) intersect at a point of intersection and the first and second focus zone (F1 and F2 respectively) coincide substantially and wherein by means of the generator device (28) the shock wave source (1a,1b,1c) and the ultrasound source (5) are able to be driven at the same time as well as alternating temporally successively.

2. A device according to claim 1, characterised in that an ultrasound locating device (5,8,46,47,52a,52b,54) is provided for locating concretions (27) to be shattered, which device has an ultrasound applicator (5,8) able to be coupled acoustically to the body (26) of the living organism and means (46,47,54) and able to be connected to this, means for generating ultrasound images, wherein ultrasound images at least of the region surrounding the point of intersection of the acoustic axes (1a,1b,1c and 6) of the shock wave source (1a,1b,1c) and of the ultrasound source (5) can be generated.

3. A device according to claim 2, characterized in that an ultrasound-sector-applicator is provided as ultrasound applicator (8), which is arranged such that a plane containing the acoustic axis (4a,4b,4c) of the shock wave source (1a,1b,1c) and/or the acoustic axis (6) of the ultrasound source (5) is able to be scanned.

4. A device according to claim 2 or 3, characterised in that the ultrasound source (5) is provided as ultrasound applicator, which is able to be connected optionally to the means (46,54) for generating ultrasound images or to the generator device (28).

5. A device according to one of claims 1 to 4, characterised in that the shock wave source (1a,1b,1c), the ultrasound source (5) and possibly the ultrasound applicator (8) are attached to a common carrier part (14).

6. A device according to one of claims 1 to 5, characterised in that the generator device (28) has a first generator (29,29a,29b), by means of which the shock wave source (1a,1b,1c) is able to be driven, a second generator (30), by means of which the ultrasound source (5) is able to be driven and control means (35), cooperating with the generators (29,29a,29b,30), wherein the first generator (29,29a,29b) has a trigger input (33,33a,33b) by way of which it is able to be activated by means of a trigger pulse (I,Ia,Ib) such that it drives the shock wave source (1a,1b,1c) to deliver a shock wave (SWa,SWb,SWc), when a trigger pulse (I,Ia,Ib) is present, wherein the second generator (30) has a control input (34) by way of which it is able to be activated by means of a control pulse (S), such that for the pulse length (b) of the control pulse (s) it drives the ultrasound source (5) to deliver ultrasound waves (USW), and wherein the control means (35) has means (36,39,39a,39b) for generating the trigger pulse (I,Ia,Ib) and means (42,45,U) for generating the control pulse (S), and the means (36,39,39a,39b) for generating the trigger pulse (I,Ia,Ib) has a pulse generator (36) which delivers periodic pulses (T) the period duration (t) of which is able to be adjusted.

7. A device according to claim 6, characterised in that the means (42,45,U) for generating the control pulse (S) according to an embodiment of the invention has a control pulse generator (42) which delivers control pulses (S), the pulse length (b) of which is able to be adjusted and which has an input (44), by way of which it is able to be activated by means of a pulse (T), in that it delivers a control pulse (S) when a pulse (T) is present, and in that the control means (35) for generating the pulse (T) for the input (44) of the control pulse generator (42) has a pulse generator (36), which generates periodic pulses (T), the period (t) of which is able to be adjusted.

8. A device according to claim 7, characterised in that the means (36,39,39a,39b) for generating the trigger pulse (I,Ia,Ib) and the means (42, 45, U) for generating the control pulse (S) have a common pulse generator (36).

9. A device according to one of claims 6 to 8, characterised in that means (36,39,39a,39b) for generating the trigger pulse (I,Ia,Ib) has a pulse delay circuit (39,39a,39b) connected between the pulse generator (36) and the trigger input (33,33a,33b) of the first generator (29,29a,29n) for delaying the pulses (T) supplied by the pulse generator (36), the pulse delay time (ta,tb) of which is able to be adjusted.

10. A device according to one of claims 2 to 9, characterised in that the control means (35) has a control circuit (49) allocated to the ultrasound locating device (5,8,46,47,52a,52b,54) and the ultrasound locating device (5,8,46,47) is able to be activated by means of an activation pulse (A) for generating ultrasound images, wherein the control circuit (49) generates the activation pulse (A) and is formed so that it only generates an activating pulse (A) when neither the first generator (29,29a,29b) is driving the shock wave source (1a,1b,1c) nor the second generator (30) the ultrasound source (5).

## Revendications

1. Dispositif pour fragmenter sans contact des concrétions (27) dans le corps (26) d'un être vivant à l'aide d'ondes acoustiques focalisées (SWa, SWb, SWc, USW), comportant une source d'ondes de choc (1a,1b,1c) servant à produire des ondes de choc (SWa,SWb,SWc), qui convergent vers une première zone locale (F1) située sur l'axe acoustique (4a,4b,4c) de la source d'ondes de choc (1a,1b,1c), une source d'ultrasons servant à produire des ondes ultrasonores (USW), qui convergent vers une seconde zone locale (F2) située sur l'axe acoustique (6) de la source d'ultrasons (5), un dispositif générateur (28), au moyen duquel la source d'ondes de choc (1a,1b,1c) et la source d'ultrasons (5) peuvent être commandées, et des moyens (25) de couplage acoustique du dispositif au corps (26) de l'être vivant, à l'aide desquels aussi bien les ondes de choc (SWa, SWb, SWc) que les ondes ultrasonores (USW) peuvent être introduites dans le corps (26) de l'être vivant pour appliquer des contraintes mécaniques aux concrétions, la source d'ondes de choc (1a,1b,1c) et la source d'ultrasons (5) étant disposées fixes l'une par rapport à l'autre de sorte que leurs axes acoustiques (4a,4b,4c,6) se coupent en un point d'intersection et que les première et seconde zones locales (F1 et F2) coïncident sensiblement, la source d'ondes de choc (1a,1b,1c) et la source d'ultrasons (5) pouvant être commandées aussi bien simultanément que d'une manière alternée, successivement dans le temps, par le dispositif générateur (28).

2. Dispositif suivant la revendication 1, caractérisé par le fait qu'il est prévu un dispositif de repérage ultrasonique (5,8,46,47,52a,52b,54) servant à localiser des concrétions (27) devant être fragmentées et qui comporte un applicateur d'ultrasons (5,8), qui peut être couplé acoustiquement au corps (26) de l'être vivant, et des moyens (46,47,54) pouvant être connectés à cet applicateur et servant à produire des images formées par ultrasons, ce qui permet d'obtenir des images formées par ultrasons au moins de la zone qui entoure le point d'intersection des axes acoustiques (1a,1b et 6) de la source d'ondes de choc (1a,1b, 1c) et de la source d'ultrasons (5).

3. Dispositif suivant la revendication 2, caractérisé par le lait qu'il est prévu comme applicateur ultrasonique (8), un applicateur ultrasonique à secteurs, qui est disposé de telle sorte qu'un plan contenant l'axe acoustique (4a,4b,4c) de la source d'ondes de choc (1a,1b,1c) et/ou l'axe optique (6) de la source d'ultrasons (5), peut être exploré.

4. Dispositif suivant la revendication 2 ou 3, caractérisé par le fait qu'il est prévu comme applicateur ultrasonique, la source d'ultrasons (5), qui peut être raccordée au choix aux moyens (46,54) servant à produire des images formées par ultrasons ou au dispositif générateur (28).

5. Dispositif suivant l'une des revendications 1 à 4, caractérisé par le lait que la source d'ondes de choc (1a,1b,1c), la source d'ultrasons (5) et éventuellement l'applicateur ultrasonique (8) sont disposés sur une partie de support commune (14).

6. Dispositif suivant l'une des revendications 1 à 5, caractérisé par le fait que le dispositif générateur (28) possède un premier générateur (29,29a,29b), à l'aide duquel la source d'ondes de choc (1a,1b,1c) peut être commandée, un second générateur (30), au moyen duquel la source d'ultrasons (5) peut être commandée, et des moyens de commande (35) coopérant avec les générateurs (29,29a,29b,30), le premier générateur (29,29a,29b) possédant une entrée de déclenchement (33,33a,33b), au moyen de laquelle il peut être activé à l'aide d'une impulsion de déclenchement (I,Ia,Ib) de sorte qu'il commande la source d'ondes de choc (1a,1b,1c), dans le cas de la présence d'une impulsion de déclenchement (I,Ia,Ib) pour la délivrance d'une onde de choc (SWa,SWb,SWc), le second générateur (30) possédant une entrée de commande (34), au moyen de laquelle il peut être activé à l'aide d'une impulsion de commande (S) de manière à commander, pendant la durée (b) de l'impulsion de commande (S), la source d'ultrasons (5) pour qu'elle délivre des ondes ultrasonores (USW), tandis que les moyens de commande (35) possèdent des moyens (36,39,39a,39b) servant à produire l'impulsion de déclenchement (I,Ia,Ib) et des moyens (42,45,U) servant à produire l'impulsion de commande (S) et que les moyens (36,39,39a,39b) servant à produire l'impulsion de déclenchement (I,Ia,Ib) possèdent un générateur d'impulsions (36), qui délivre des impulsions périodiques (N) de période (t) réglable.

7. Dispositif suivant la revendication 6, caractérisé par le lait que les moyens (42,45,U) servant à produire l'impulsion de commande (S) possèdent, conformément à une forme de réalisation de l'invention, un générateur d'impulsions de commande (42), qui délivre des impulsions de commande (S), de longueur (b) est réglable, et possède une entrée (44), au moyen de laquelle il peut être activé à l'aide d'une impulsion (T) de telle sorte que, dans le cas de la présence d'une impulsion (T), il délivre une impulsion de commande (S), et que les moyens de commande (35) servant à produire l'impulsion (T) pour l'entrée (44) du générateur d'impulsions de commande (42) possèdent un générateur d'impulsions (36), qui produit des impulsions périodiques (T) de période (t) réglable.

8. Dispositif suivant la revendication 7, caractérisé par le fait que les moyens (36,39,39a,39b) servant à produire l'impulsion de déclenchement (I,Ia,Ib) et les moyens (42,45,U) servant à produire l'impulsion de commande (S) possèdent un générateur d'impulsions commun (36).

9. Dispositif suivant l'une des revendications 6 à 8, caractérisé par le lait que les moyens (36,39,39a,39b) servant à produire l'impulsion de déclenchement (I,Ia,Ib) possèdent un circuit de retardement d'impulsions (39,39a,39b), qui est branché entre le générateur d'impulsions (36) et l'entrée de déclenchement (33,33a,33b) du premier générateur (29,29a,29b) et qui sert à retarder les impulsions (T) qui sont délivrées par le générateur d'impulsions (36) et dont le retard (ta,tb) est réglable.

10. Dispositif suivant l'une des revendications 2 à 9, caractérisé par le fait que les moyens de commande (35) possèdent un circuit de commande (49) associé au dispositif de repérage ultrasonique (5,8,46,47,52a,52b,54), et que le dispositif de repérage ultrasonique (5,8,46,47) peut être activé au moyen d'une impulsion d'activation (A) servant à produire des images formées par ultrasons, le circuit de commande (49) produisant l'impulsion d'activation (A) et étant agencé de telle sorte qu'il produit une impulsion d'activation (A) uniquement lorsque ni le premier générateur (29,29a,29b) ne commande la source d'ondes de choc (1a,1b,1c), ni le second générateur (30) ne commande la source d'ultrasons (5).
